# EUROPÄISCHE PATENTSCHRIFT

(11) **EP 3 131 108 B1**
(45) Veröffentlichungstag und Bekanntmachung des Hinweises auf die Patenterteilung: **10.10.2018**
(21) Anmeldenummer: 15181044.7
(22) Anmeldetag: 14.08.2015
(51) Int. Cl.: H01H 11/00, H01H 13/803, H01H 13/807, A61M 15/00, H01H 3/16, H01H 3/38, H01H 9/02

(54) **ELEKTROMECHANISCHE SCHALTVORRICHTUNG**
ELECTROMECHANICAL SWITCHING DEVICE
DISPOSITIF DE COMMUTATION ELECTROMECANIQUE

(43) Veröffentlichungstag der Anmeldung: 15.02.2017
(73) Patentinhaber: RAFI GmbH & Co. KG, 88276 Berg (DE)
(72) Erfinder: WASMEIER, Albert, 88677 Markdorf (DE)
(74) Vertreter: Engelhardt, Volker

(56) Entgegenhaltungen:
- DE-A1- 10 151 053
- DE-B- 1 190 544
- DE-T2- 3 850 936
- US-A- 5 743 252
- US-A1- 2004 168 899
- US-B1- 7 950 970

## Beschreibung

Die Erfindung bezieht sich auf eine elektromechanische Schaltvorrichtung zur Erzeugung von mindestens zwei unabhängigen elektrischen Schaltsignalen durch eine Betätigung nach dem Oberbegriff des Patentanspruches 1 sowie auf ein Verfahren zur Herstellung einer derartigen Schaltvorrichtung nach dem Oberbegriff des Patentanspruches 10.
Zur Bedienung von Spraydosen, insbesondere von Spraydosen in denen ein Asthmamedikament eingefüllt ist, ist es oftmals erforderlich, mindestens zwei Schaltsignale durch unabhängig voneinander einwirkende Betätigungskräfte zur definierten Ausströmung des Medikamentes aus der Spraydose zu erzeugen. Die Spraydose wird nämlich in eine Hosentasche oder in sonstige Behältnisse gesteckt, so dass durch Bewegungen oder unbeabsichtigte äußere Krafteinwirkungen die Spraydose betätigt wird, wodurch die teure Medikation ungenutzt aus der Spraydose ausgebracht ist.
Um diese Nachteile zu beheben, sind zwei unabhängig voneinander zu betätigende Schalter vorzusehen, die nacheinander zu aktivieren sind. Darüber hinaus ist es erforderlich, dass durch eine elektrische Auswerteeinrichtung festgestellt wird, dass tatsächlich aus dem Vorratsraum der Spraydose in einen Zwischen-Lagerraum der Spraydose das Medikament dosiert eingefüllt ist und dass nachfolgend das Ausbringen des Medikamentes aus dem Lagerraum erfolgt.

US 2004/168899 offenbart bzl. Abb. 8 eine elektromechanische Schaltvorrichtung zur Erzeugung von mindestens zwei unabhängigen elektrischen Schaltsignalen durch eine Betätigung bestehend aus einem Gehäuse, aus mindestens zwei räumlich voneinander beabstandeten Schaltern die die von außerhalb des Gehäuses auftretenden jeweiligen Betätigungskräfte aufnehmen das mindestens zwei parallel zueinander verlaufende Stränge aufweisen wobei der erste Schalter als Öffner und der zweite Schalter als Schließer ausgestaltet sind und dass das Gehäuse aus einem Kunststoff gefertigt ist. Es ist daher Aufgabe der Erfindung eine Schaltvorrichtung, insbesondere für die Bedienung von solchen Spraydosen, zur Verfügung zu stellen, die möglichst kostengünstig herstellbar und gleichzeitig eine zuverlässige und dauerhafte Bedienbarkeit der Spraydose gewährleistet. Zudem soll durch die Schaltvorrichtung eine elektrische Auswerteeinrichtung angesteuert sein, um die Dosierung des Medikamentes sowie die Anzahl der Sprayvorgänge der Spraydose einstellen bzw. überwachen zu können.

Diese Aufgaben sind erfindungsgemäß durch eine elektromechanische Schaltvorrichtung der eingangs genannten Gattung sowie ein Verfahren für die Herstellung einer solchen Schaltvorrichtung nach den kennzeichnenden Teilen der Patentansprüche 1 und 10 gelöst.

Weitere vorteilhafte Weiterbildungen der Erfindung ergeben sich aus den Unteransprüchen.

Dadurch, dass die einzelnen Stränge (4) aus einem einzelnen plattenförmigen Bauteil herausgestanzt sind, dessen Werkstoff elektrisch leitfähig ist, wobei das Bauteil als ein Stanzgitter (3) ausgebildet ist, dass das Gehäuse (2) die einzelnen Stränge (4) des Stanzgitters (3) ummantelt, und dass das Gehäuse (2) aus einem Kunststoff gefertigt ist, soll ein Schalter nämlich als Öffner und der andere Schalter als Schließer wirken. Das bedeutet, dass durch die auftretende äußere Betätigungskraft der Schalter von einer permanenten Schließstellung zweier Stränge in eine geöffnete Stellung überführt ist und dass der jeweilige Schließer aus einer angehobenen Position in eine geschlossene demnach verbundene Position zweier Stränge durch Einwirkung einer unabhängigen weiteren Betätigungskraft überführt ist und diese jeweiligen Schaltvorgänge dazu führen, dass eine permanente Spannungsversorgung durch den Öffner unterbrochen ist und durch den Schließer wiederhergestellt ist. Diese Schaltungsvorgänge kann die Auswerteeinrichtung feststellen und demnach ein entsprechendes Schaltsignal erzeugen, wenn diese Schaltvorgänge zeitlich nachfolgend ablaufen.

Insbesondere eine Spraydose, in der ein Medikament eingefüllt ist, kann mittels dieser erfindungsgemäßen Schaltvorrichtung zuverlässig und von außen überwacht betrieben werden. Denn zum einen kann mittels der Auswerteeinrichtung die Dosierung des in der Spraydose eingefüllten Medikamentes eingestellt und zum anderen die Anzahl der Sprayvorgänge pro Zeiteinheit festgestellt bzw. begrenzt sein.

Darüberhinaus ist besonders vorteilhaft, dass die elektromechanische Schaltvorrichtung aus einem Stanzgitter hergestellt ist, das mit relativ geringen Fertigungskosten herstellbar ist. Zudem kann das Stanzgitter in einem Kunststoffspritzgussverfahren teilweise ummantelt werden, um dieses entsprechend zu kapseln und vor Verunreinigungen zu schützen.

Da das Stanzgitter aus einem elektrisch leitfähigen Werkstoff besteht, dient dieses gleichzeitig als elektrische Zuleitung für die Auswerteeinrichtung, die beispielsweise mit einer internen oder externen Stromquelle zur Spannungsversorgung verbunden ist.

In der Zeichnung ist die erfindungsgemäße elektromechanische Schaltvorrichtung dargestellt und wird nachfolgend näher erläutert. Im Einzelnen zeigt:
- Figuren 1a und 1b: die Verfahrensschritte zur Herstellung der elektromechanischen Schaltvorrichtung, bestehend aus einem Stanzgitter mit vier parallel zueinander verlaufenden Strängen, an denen räumlich zueinander benachbart zwei Schalter angebracht sind,
- Figur 2: die Schaltvorrichtung gemäß Figur 1b, in perspektivischer Ansicht mit einem Öffner-Schalter und einem Schließer-Schalter sowie mit einem die Stränge ummantelnden Gehäuse aus Kunststoff und
- Figur 3: ein Anwendungsbeispiel der Schaltvorrichtung gemäß Figur 2 in einer Spraydose, im Schnitt.

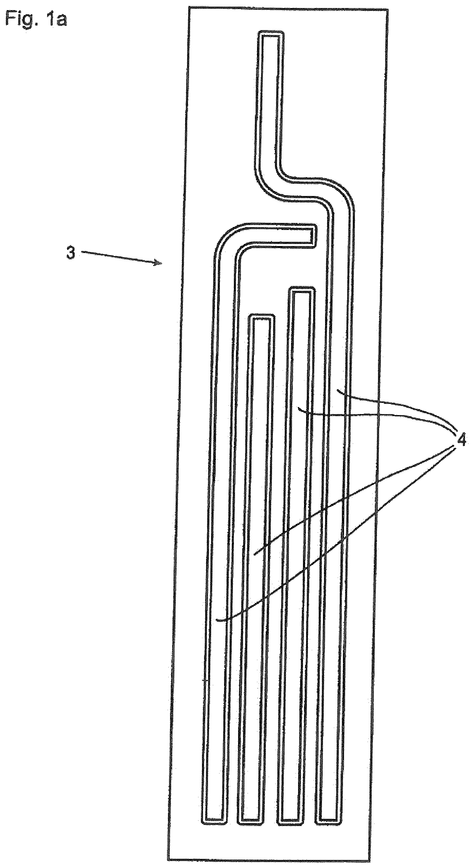

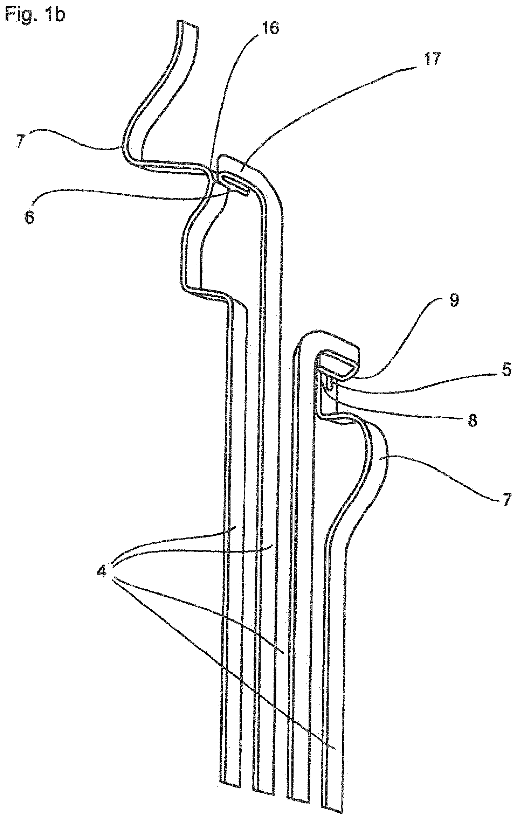

In den Figuren 1a und 1b sind die Herstellungsschritte zur Fertigung einer elektromechanischen Schaltvorrichtung 1 abgebildet, die im Wesentlichen aus einem Gehäuse 2 und einem Stanzgitter 3 besteht. Das Stanzgitter 3 weist vier einzelne Stränge 4 auf, die aus einem plattenförmigen Bauteil herausgestanzt sind. Das Stanzgitter 3 ist aus einem elektrisch leitfähigen Werkstoff gebildet, das beispielsweise an eine Stromquelle, einen Akkumulator oder an eine andersartig ausgestaltete Spannungsversorgung angeschlossen werden kann. Die vereinzelten Stränge 4 verlaufen parallel zueinander.

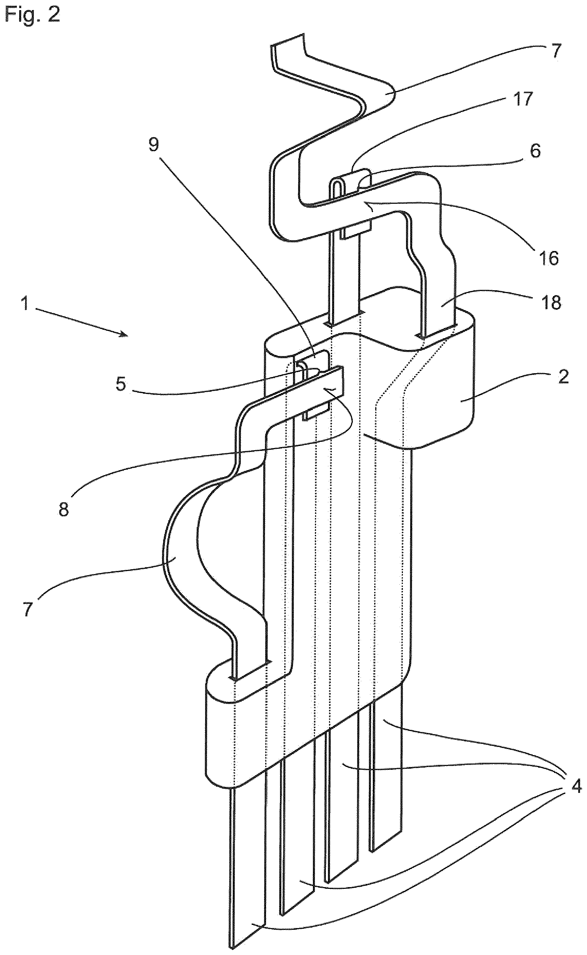

Insbesondere den Figuren 1b und 2 ist zu entnehmen, dass zwei der vier Stränge 4 des Stanzgitters 3 zu einem Kontaktbügel 7 umgebogen sind. Der Kontaktbügel 7 des ersten Schalters 5 wirkt dabei als Öffner und der zweite Kontaktbügel 7 des Schalters 6 als Schließer. Darunter ist zu verstehen, dass der Öffner-Schalter 5 permanent, beispielsweise aufgrund von Vorspannkräften in Form einer Feder ausgestalteten Kontaktbügels 7, an zwei Positionen 8 und 9 elektrisch mit dem jeweiligen Strang 4 des Stanzgitters 3 gekoppelt ist und der Schließer-Schalter 6 lediglich an einer Position 16 mit dem Strang 4 des Stanzgitters 3 verbunden ist und an der gegenüberliegenden Position 18 von dem Strang 4 des Stanzgitters 3 abgehoben ist.

Die Ausgestaltung des jeweiligen Kontaktbügels 7 kann wahlweise derart ausgestaltet sein, dass durch diesen entweder ein geschlossener Stromkreis über einen der Stränge 4 oder über zwei benachbarte Stränge 4 gebildet ist.

Die einzelnen Stränge 4 des Stanzgitters 3 werden in einem Kunststoffspritzgussverfahren gemäß Figur 2 ummantelt, so dass diese gekapselt sind. Innerhalb des Gehäuses 2, das aus dem Kunststoffwerkstoff gefertigt ist, können elektrische Bauteile, beispielsweise Mikroprozessoren oder sonstige elektrische Auswerteeinrichtungen 10 angeordnet und elektrisch mit dem jeweiligen Strang 4 des Stanzgitters 3 verbunden sein. Sobald eine Betätigungskraft auf den Öffner-Schalter 5 einwirkt, wird dieser an der Position 9 von dem Strang 4 des Stanzgitters 3 abgehoben. Die elektrische Verbindung am Punkt 8 zwischen der Spannungsversorgung und der Auswerteeinrichtung 10 ist somit bei der Betätigung des Öffner-Schalters 5 unterbrochen, wodurch ein elektrisches Schaltsignal erzeugt ist, das von der elektrischen Auswerteeinrichtung 10 erfasst und in ein entsprechendes Schaltsignal umgewandelt ist.

Der Schließer-Schalter 6 funktioniert umgekehrt, denn wenn auf diesen eine Betätigungskraft einwirkt, wird der Kontaktbügel 7 des Schließer-Schalters 6 an der abgehobenen Position 16 in Richtung des Stranges 4 des Stanzgitters 3 gedrückt, so dass der Kontaktbügel 7 des Schließer-Schalters 6 geschlossen ist, wodurch die elektrische Spannung zwischen der Spannungsversorgung und der Auswerteeinrichtung 10 überbrückt ist. Auch dieser Schaltvorgang ist von der elektrischen Auswerteeinheit 10 erfasst und in ein elektrisches Schaltsignal umgewandelt.

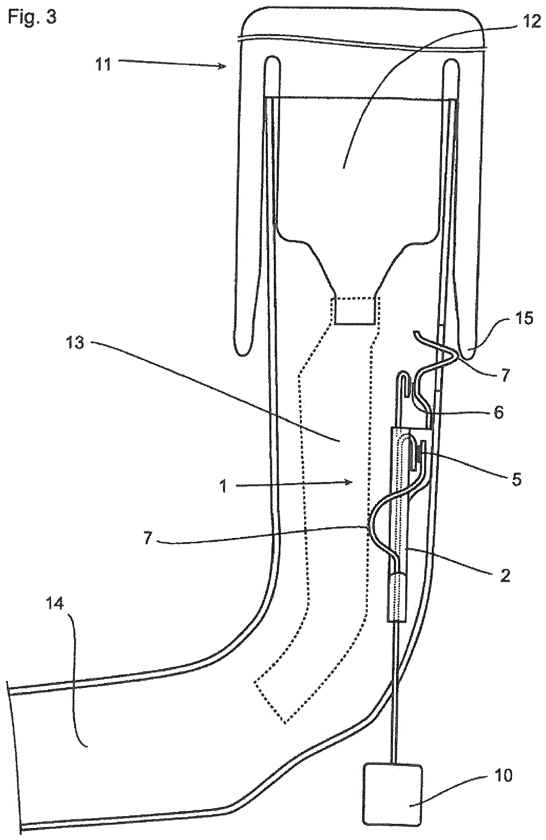

Ein mögliches Anwendungsbeispiel ist der Figur 3 zu entnehmen. Dort ist die Schaltvorrichtung 1 in eine Spraydose 11 mit einem Vorratsraum 12 und einem Lagerraum 13, die räumlich voneinander getrennt sind, eingebaut. In den Vorratsraum 12 kann beispielsweise ein Medikament zur Behandlung von Atemwegserkrankungen, beispielsweise Asthma, eingefüllt sein.

Der Schließer-Schalter 6 der elektromechanischen Schaltvorrichtung 1 ist dabei einem von außen zugänglichen und manuell betätigbaren Druckstück 15 zugeordnet. Sobald auf das Druckstück 15 eine Betätigungskraft auf den Schließer-Schalter 6 aufgebracht ist, ist durch die Auswerteeinheit 10 ein elektrisches Schaltsignal erzeugt, wodurch aus dem Vorratsraum 12 in den Lagerraum 13 das eingefüllte Medikament ausgebracht ist. Dem Lagerraum 13 ist der Öffner-Schalter 5 zugeordnet und mittels einer Membran-Folie oder einem sonstigen elastisch verformbaren Dichtungselement abgedeckt. Durch den Druckaufbau in dem Lagerraum 13 wird von dem Medikament auf den Öffner-Schalter 5 ein Druck- bzw. eine dadurch hervorgerufene Betätigungskraft aufgebracht und der Kontakt geöffnet, wodurch sich ein elektrisches Schaltsignal durch die Auswerteeinrichtung 10 erzeugen lässt. Durch dieses Schaltsignal wird die in dem Lagerraum 13 eingebrachten Medikamente in Richtung des Mundstückes 14 der Spraydose 11 ausbracht, so dass der Patient das Medikament einatmen kann.

Durch die beiden räumlich voneinander getrennten Öffner 5 und Schließer 6 ist vorteilhafterweise gewährleistet, dass ausschließlich bei deren chronologischen Bedienung das Medikament aus der Spraydose 11 ausströmt. Sollte beispielsweise der Öffner 5 betätigt sein, strömt kein Medikament aus, denn dieses ist im Lagerraum 13 erst dann eingefüllt, wenn bewusst das Druckstück 15 und somit der Schließer 6 betätigt wurde.

Darüberhinaus kann die elektrische Auswerterichtung 10 derart programmiert sein, dass innerhalb eines vorgegebenen Zeitraumes eine bestimmte Menge des Medikamentes für den jeweiligen Patienten zur Verfügung gestellt wird. Ist die vorgegebene Dosierung überschritten, kann das Druckstück 15 bzw. der Öffner-Schalter 5 deaktiviert werden, so dass der Patient keine weitere Medikamentierung bis zum Ablauf des vorgegebenen Zeitintervalls vornehmen kann. Desweiteren ist es möglich durch die Programmierung der Auswerteeinrichtung 10 die Dosierung des Medikamentes über ein bestimmten Zeitraum zu überwachen und exakt zu bestimmen, wieviel des Medikamentes von dem Patienten eingeatmet bzw. aus der Spraydose 11 ausgebracht wurde.

Die erfindungsgemäße Schaltvorrichtung 1 hat demnach nicht nur die Aufgabe eine möglichst zuverlässige und chronologisch abgestimmte Schaltreihenfolge zur Verfügung zu stellen, sondern vielmehr auch die jeweils für den Patienten erforderliche Medikamentierung zu überwachen, zu regeln und exakt einstellen zu können.

Der Kontaktbügel 7 kann dabei unterschiedlichste Konturen aufweisen, um eine entsprechende Vorspannkraft, die in Richtung der Stränge 4 des Stanzgitters 3 oder entgegengesetzt dazu wirkt, aufzuweisen. Der Kontaktbügel 7 des Öffner-Schalters 5 ist nämlich aufgrund seiner Vorspannkraft permanent an den beiden Positionen 8 und 9 mit dem jeweiligen Strang 4 des Stanzgitters 3 verbunden. Durch die auf den Öffner-Schalter 5 einwirkende Betätigungskraft wird die Vorspannung des Kontaktbügels 7 überwunden, da die Betätigungskraft größer bemessen ist, als die Vorspannkraft des Betätigungsbügels 7 des Öffner-Schalters 5.

Der Schließer-Schalter 6 weist ein Vorspannkraft auf, die entgegengesetzt zu dem Stanzgitter 3 verläuft, so dass durch die einwirkende Betätigungskraft der Kontaktbügel 7 des Schließer-Schalters 6 in Richtung des jeweiligen Stranges 4 des Stanzgitters 3 gedrückt ist.

Der jeweilige Öffner 5 und Schließer 6 ist außerhalb des Gehäuses 2 angeordnet, so dass die Bewegungen des Kontaktbügels 7 durch dieses nicht behindert sondern freigegeben sind.

Darüberhinaus ist es möglich, den jeweiligen Kontaktbügel 7 als separates Bauteil vorzusehen und an der Position 18 mit dem Stanzgitter 3 zu verkleben, zu verschweißen oder zu verlöten. Die Konturen des Kontaktbügels 7 sind dabei wellenförmig, V-förmig, bogenförmig und/oder konkav bzw. konvex gekrümmt ausgestaltet.

## Patentansprüche

1. Elektromechanische Schaltvorrichtung zur Erzeugung von mindestens zwei unabhängigen elektrischen Schaltsignalen durch eine Betätigung, insbesondere zur Bedienung einer mit einem Medium gefüllten Spraydose (11), bestehend aus einem Gehäuse (2), aus mindestens zwei räumlich voneinander beabstandeten Schaltern (5, 6), die die von außerhalb des Gehäuses (2) auftretenden jeweiligen Betätigungskräfte aufnehmen, aus einem Gitter, das mindestens zwei parallel zueinander verlaufende Stränge (4) aufweist, und aus einer elektrischen Auswerteeinrichtung, die elektrisch mit den Schaltern (5, 6) und den vereinzelten Strängen (4) gekoppelt und durch die die Schalter (5, 6) mittels einer Spannungsversorgung überwacht sind, wobei der erste Schalter als Öffner (5) und der zweite Schalter als Schließer (6) ausgestaltet sind, wobei die einzelnen Stränge (4) aus einem einzelnen plattenförmigen Bauteil herausgestanzt sind, dessen Werkstoff elektrisch leitfähig ist, wobei das Bauteil als ein Stanzgitter (3) ausgebildet ist,
das Gehäuse (2) die einzelnen Stränge (4) des Stanzgitters (3) ummantelt,
und das Gehäuse (2) aus einem Kunststoff gefertigt ist.

2. Schaltvorrichtung (1) nach Anspruch 1,
**dadurch gekennzeichnet,**
**dass** der Öffner (5) und der Schließer (6) aus einem Kontaktbügel (7) gebildet sind, dass der Kontaktbügel (7) des Öffners (5) durch eine eigene Vorspannkraft permanent mit dem Stanzgitter (3) an zwei voneinander beabstandeten Positionen (8, 9) verbunden ist, dass der Kontaktbügel (7) des Schließers (6) an einer ersten Position (17) fest mit dem Stanzgitter (3) verbunden ist und dass die Vorspannung des Kontaktbügels (7) des Schließers (6) dessen Abheben von dem Stanzgitter (3) an der zweiten gegenüberliegenden Position (16) bewirkt.

3. Schaltvorrichtung (1) nach Anspruch 2,
**dadurch gekennzeichnet,**
**dass** durch die Betätigungskraft der Kontakt des Öffners (5) von dem Stanzgitter (3) an der Position (9) zur Unterbrechung der elektrischen Verbindung abgehoben ist.

4. Schaltvorrichtung (1) nach Anspruch 2,
**dadurch gekennzeichnet,**
**dass** der Kontaktbügel (7) des Schließers (6) von dem Stanzgitter (3) im unbetätigten Zustand an einer der Positionen (17) abgehoben ist und dass durch eine zweite Betätigungskraft der Schließer (6) an dieser Position (16) mit dem Stanzgitter (3) elektrisch gekoppelt ist, sobald eine entsprechend groß bemessene Betätigungskraft auf diesen einwirkt.

5. Schaltvorrichtung (1) nach einem der Ansprüche 2 bis 4,
**dadurch gekennzeichnet,**
**dass** der jeweilige Kontaktbügel (7) aus einem der Stränge (4) des Stanzgitters (3) durch Umformen oder Umbiegen gebildet ist, oder dass der jeweilige Kontaktbügel (7) als separates Bauteil mit einem der Stränge (4) des Stanzgitters (3) durch Verlöten, Verkleben oder Verschweißen fest an der Position (18) verbunden ist.

6. Schaltvorrichtung (1) nach einem der vorgenannten Ansprüche,
**dadurch gekennzeichnet,**
**dass** in dem Gehäuse (2) Durchgangsöffnungen vorgesehen sind, die fluchtend zu dem jeweiligen Schalter (5, 6) angeordnet sind, dass in der jeweiligen Durchgangsöffnung eine der das Stanzgitter (3) bildenden Stränge (4) verläuft, an dem der jeweilige Schalter (5, 6) vorgesehen ist.

7. Schaltvorrichtung (1) nach einem der vorgenannten Ansprüche 2 bis 6,
**dadurch gekennzeichnet,**
**dass** die Ausbildung des Kontaktbügels (7) des jeweiligen Schalters (5, 6) dom-, V- oder bogenförmig bzw. konkav oder konvex gekrümmt ausgestaltet ist und dass der Kontaktbügel (7) eine Federkraft aufweist, die in Abhängigkeit von der Ausgestaltung des Kontaktbügels (7) eine Betätigungskraft entgegenwirkt.

8. Schaltvorrichtung (1) nach Anspruch 7,
**dadurch gekennzeichnet,**
**dass** die Federkraft des jeweiligen Kontaktbügels (7) vorgegeben und der jeweiligen ausgeübten Betätigungskraft auf den Schalter (5, 6) entgegenwirkt.

9. System beinhaltend eine Spraydose und eine Schaltvorrichtung (1) nach einem der vorgenannten Ansprüche,
**dadurch gekennzeichnet,**
**dass** die Schaltvorrichtung (1) an oder in einer Spraydose (11) befestigt oder angeordnet ist, dass durch den Schließer-Schalter (6) aus einem Vorratsraum (12) der Spraydose (11) das Medium in einen Lagerraum (13), der in der Spraydose (11) eingearbeitet ist, ausgebildet ist, dass der Öffner-Schalter (5) dem Lagerraum (13) zugeordnet ist und dass der in dem Lagerraum (13) nach dem Einfüllen des Mediums aus dem Vorratsraum (12) ein Druck aufgebaut ist, durch den der Öffnerschalter (5) betätigt ist, derart, dass das in dem Lagerraum (13) vorhandene Medium, aus der Spraydose (11) ausströmt.

10. Verfahren zur Herstellung einer Schaltvorrichtung (1), insbesondere nach einem der vorgenannten Ansprüche, beinhaltend die nachfolgend aufgeführten Verfahrensschritte:
- Anfertigen eines Stanzgitters (3) aus einem plattenförmigen Bauteil, das aus einem elektrisch leitfähigen Werkstoff besteht, derart, dass mindestens zwei Stränge (4), nach dem Stanzvorgang entstehen,
- Ausbilden von mindestens zwei Schaltern (5, 6) an einem der Stränge (4) des Stanzgitter (3) durch Umbiegen der freien Enden der Stränge (4) oder durch Aufschweißen eines Kontaktbügels (7), wobei einer der Schalter als Öffner (5) und der andere Schalter als Schließer (6) ausgestaltet ist,
- und
- Umspritzen des Stanzgitters (3) in einem Spritzguss-Kunststoffverfahren zur Bildung eines Gehäuses (2), derart, dass die Schalter (5, 6) außerhalb des Gehäuses (2) angeordnet sind und die Stränge (4) durch das Gehäuse (2) ummantelt sind.

## Claims

1. An electromechanical switching device for generating at least two independent electrical switching signals through one actuation, in particular for operating a spray can (11) filled with a medium, consisting of a housing (2), of at least two switches (5, 6) that are spatially separated from one another and are acted upon by actuation forces from outside the housing (2) in each case, of a grid with at least two strands (4) running parallel to one another, and of an electrical evaluation device which is electrically coupled to the switches (5, 6) and the individual strands (4) and by means of which the switches (5, 6) are monitored by a voltage supply, in which case the first switch is configured as a normally closed contact (5) and the second switch as a normally open contact (6),
**characterised in that**
the individual strands (4) are punched out of a single plate-shaped component, the material of which is electrically conductive, in which case the component is configured as a punched grid (3),
the housing (2) surrounds the individual strands (4) of the punched grid (3), and the housing (2) is produced from a polymer.

2. The switching device (1) in accordance with Claim 1,
**characterised in that**
the normally closed contact (5) and the normally open contact (6) are formed from a contact bar (7), that the preload force of the contact bar (7) of the normally closed contact (5) holds it permanently connected to the punched grid (3) at two positions (8, 9) which are spaced apart, that the contact bar (7) of the normally open contact (6) is firmly connected to the punched grid (3) at one first position (17) and that the preload on the contact bar (7) of the normally open contact (6) causes it to be lifted off the punched grid (3) at the second, opposite position (16).

3. The switching device (1) in accordance with Claim 2,
**characterised in that**
the actuation force causes the contact of the normally closed contact (5) to be lifted off the punched grid (3) at the position (9) in order to interrupt the electrical connection.

4. The switching device (1) in accordance with Claim 2,
**characterised in that**
the contact bar (7) of the normally open contact (6) is lifted off the punched grid (3) in the non-actuated condition at one of the positions (17) and that a second actuation force causes the normally open contact (6) to be electrically coupled with the punched grid (3) at this position (16) as soon as an actuation force of a corresponding magnitude acts upon it.

5. The switching device (1) in accordance with Claims 2 to 4,
**characterised in that**
the corresponding contact bar (7) is formed from one of the strands (4) of the punched grid (3) by the process of forming or bending, or that the corresponding contact bar (7) is a separate component fixedly connected to one of the strands (4) of the punched grid (3) by soldering, gluing or welding, at the position (18).

6. The switching device (1) in accordance with one of the aforementioned claims,
**characterised in that**
passage openings are provided in the housing (2) which are arranged flush with the corresponding switch (5, 6), that one of the strands (4) forming the punched grid (3) runs within the corresponding passage opening and the particular switch (5, 6) is provided on it.

7. The switching device (1) in accordance with one of the aforementioned Claims 2 to 8,
**characterised in that**
the configuration of the contact bar (7) of the corresponding switch (5, 6) is in the shape of a dome, V or arch or it has a concave or convex curvature and that the contact bar (7) has a spring force which counteracts an actuating force depending on the configuration of the contact bar (7).

8. The switching device (1) in accordance with Claim 7,
**characterised in that**
the spring force of the corresponding contact bar (7) is specified and counteracts the actuation force that is applied in each case to the switch (5, 6).

9. A system containing a spray can and a switching device (1) in accordance with one of the aforementioned claims,
**characterised in that**
the switching device (1) is attached to or arranged in a spray can (11), that the medium is passed through the normally open contact switch (6) from a reservoir compartment (12) of the spray can (11) into a storage compartment (13) worked into the spray can (11), that the normally closed contact switch (5) is assigned to the storage compartment (13) and that after the medium has been filled from the reservoir compartment (12) a pressure is established in the storage compartment (13) by means of which the normally closed contact switch (5) is actuated in such a way that a medium existing in the storage compartment (13) flows out of the spray can (11).

10. A process for manufacturing a switching device (1) in particular in accordance with one of the aforementioned claims,
containing the process steps listed below:
- Production of a punched grid (3) from a plate-shaped component made of an electrically conductive material, in such a way that at least two strands are created after the punching procedure,
- Configuration of at least two switches (5, 6) on one of the strands (4) of the punched grid (3) by bending over the free ends of the strands (4) or by welding a contact bar (7) on, in which case one of the switches is configured as a normally closed contact (5) and the other switch as a normally open contact (6),
and
- spray moulding around the punched grid (3) in a polymer injection-moulding process for forming a housing (2), in such a way that the switches (5, 6) are arranged outside the housing (2) and the strands (4) are enclosed by the housing (2).

## Revendications

1. Dispositif de commutation électromécanique servant à la génération d'au moins deux signaux de commande électriques indépendants par un seul actionnement, notamment pour le maniement d'une bombe aérosol remplie d'un milieu (11), comprenant un boîtier (2), au moins deux commutateurs (5, 6) espacés l'un de l'autre, qui absorbent les forces d'actionnement respectives produites à l'extérieur du boîtier (2), une grille comprenant au moins deux branches (4) orientées parallèlement, et un équipement d'évaluation électrique raccordé électriquement aux commutateurs (5, 6) et aux branches individuelles (4) et qui assurent la surveillance des commutateurs (5, 6) moyennant une alimentation en tension, le premier commutateur agissant en tant que contact de repos (5) et le deuxième en tant que contact de travail (6),
où
les branches individuelles (4) sont découpées d'un seul composant en matériau conducteur sous la forme d'une plaque et où le composant est conçu sous la forme d'une grille à découpage (3), le boîtier (2) enveloppant les diverses branches (4) de la grille à découpage (3), et le boîtier (2) étant fabriqué en matière synthétique.

2. Dispositif de commutation (1) d'après la revendication 1,
**caractérisé en ce que**,
le contact de repos (5) et le contact de travail (6) sont formés par un étrier de contact (7), que, grâce à la propre pré-tension, l'étrier de contact (7) du contact de repos (5) est raccordé en permanence en deux positions espacées l'une de l'autre (8, 9) à la grille à découpage (3), que l'étrier de contact (7) du contact de travail (6) est raccordé rigidement en une première position (17) à la grille à découpage (3), et que la pré-tension de l'étrier de contact (7) du contact de travail (6) assure son soulèvement de la grille à découpage (3) en une deuxième position opposée (16).

3. Dispositif de commutation (1) d'après la revendication 2,
**caractérisé en ce que**,
par la force d'actionnement, le contact du contact de repos (5) est soulevé en position (9) de la grille à découpage (3) en vue de la coupure de la liaison électrique.

4. Dispositif de commutation (1) d'après la revendication 2,
**caractérisé en ce que**,
en état non activé, l'étrier de contact (7) du contact de travail (6) est soulevé en une des positions (17) de la grille à découpage (3) et que moyennant une deuxième force d'actionnement, le contact de travail (6) est accouplé électriquement, dans cette position (16), à la grille à découpage (3), dès qu'une force d'actionnement définie agit sur celui-ci.

5. Dispositif de commutation (1) d'après une des revendications 2 à 4,
**caractérisé en ce que**
l'étrier de contact respectif (7) est formé par reformation ou par pliage à partir d'une des branches (4) de la grille à découpage (3) ou que, en tant que composant isolé, l'étrier de contact respectif (7) est raccordé rigidement, par brasage, par collage ou par soudage, en position (18), avec une des branches (4) de la grille à découpage (3).

6. Dispositif de commutation (1) d'après une des revendications précédentes,
**caractérisé en ce que**
dans le boîtier (2), il est prévu des ouvertures de passage disposées en alignement précis avec le commutateur respectif (5, 6), que par l'ouverture de passage respective il passe une des branches (4) qui forment la grille à découpage (3) et sur laquelle il est prévu le commutateur respectif (5, 6).

7. Dispositif de commutation (1) d'après une des revendications 2 à 6,
**caractérisé en ce que**
l'étrier de contact respectif (7) du commutateur respectif (5, 6) a la forme d'un dôme, d'un V ou d'un arc, ou qu'il est concave ou convexe, et que l'étrier de contact (7) possède une force de ressort qui, en dépendance de la forme de l'étrier de contact (7) s'oppose à une force d'actionnement.

8. Dispositif de commutation (1) d'après la revendication 7,
**caractérisé en ce que**
la force de ressort de l'étrier de contact respectif (7) est définie et agit contre la force d'actionnement respective sur le commutateur (5, 6).

9. Système comprenant une bombe aérosol et un dispositif de commutation (1) d'après une des revendications précédentes,
**caractérisé en ce que**
le dispositif de commutation (1) est fixé ou arrangé sur ou dans une bombe aérosol (11), que par le contact de travail (6), le milieu est établi à partir d'une chambre de réserve (12) de la bombe aérosol (11) dans une chambre (13) pratiquée dans la bombe aérosol (11), que le contact de repos (5) est assigné à la chambre (13), et que suite au remplissage du milieu hors de la chambre de réserve (12), il est établi une pression dans la chambre (13) qui actionne le contact de repos (5) de sorte que le milieu existant dans la chambre (13) s'écoule hors de la bombe aérosol (11).

10. Procédé de fabrication d'un dispositif de commutation (1), en particulier d'après une des revendications précédentes,
comprenant
les phases de procédé suivantes :
- Préparation d'une grille à découpage (3) qui a la forme d'une plaque et qui est réalisée en matériau conducteur, de sorte qu'après le découpage, il est formé au moins deux branches (4),
- Formation d'au moins deux commutateurs (5, 6) sur une des branches (4) de la grille à découpage (3) par courbage des extrémités libres des branches (4) ou par soudage d'un étrier de contact (7), un des commutateurs étant conçu en tant que contact de repos (5) et l'autre commutateur en tant que contact de travail (6),
et
- revêtement de la grille à découpage (3) à l'aide d'un procédé de moulage par injection de matière synthétique en vue de la formation d'un boîtier (2) de sorte que les commutateurs (5, 6) se trouvent en dehors du boîtier (2) et que les branches (4) soient revêtues par le boîtier (2).
